# EUROPEAN PATENT APPLICATION

(11) **EP 1 970 604 A1**
(43) Date of publication of application: **17.09.2008**
(21) Application number: 08250897.9
(22) Date of filing: 14.03.2008
(51) Int. Cl.: F16J 15/06, F16J 13/06, F16J 15/24

(54) **Seal arrangement**

(30) Priority: 14.03.2007 GB 0704864
(71) Applicant: Blakeney Ironworks Limited, Blakeney Gloucestershire GL15 4AE (GB)
(72) Inventor: Cecil, Geoff, Blakeney, Gloucestershire GL15 4AE (GB)
(74) Representative: Bailey, Richard Alan

(57) **Abstract**

A seal arrangement comprises a seal component 22 engageable within an opening 20 formed in a housing 16, the seal component 22 defining an opening 24 through which, in use, an item to be sealed to the housing 16 extends, and a clamp device 18, 26 operable to apply a clamping load to the seal member 22, wherein the seal member 22 is of split form, a seal keystone member 32 being located in the split 30 of the seal member 22.

## Description

This invention relates to a seal arrangement, and in particular to a seal arrangement designed for use in forming a seal around a body of substantially circular or elliptical cross-section. In particular, the invention relates to a seal arrangement having a split seal member.

There are a number of situations where it is desired to form a seal between the outer surface of a body and another component. One specific application of such seals is in the sterilisation of medical or surgical equipment. Where a piece of equipment requires chemical sterilisation, but includes component parts which will be damaged by the sterilisation process, for example where the equipment includes electrical or electronic components which may be damaged by being submerged in a liquid or gas chemical sterilising agent, sterilisation is often limited to simply wiping parts of the equipment with an impregnated cloth or wipe.

To provide a better level of sterilisation, it has been proposed to locate the non-sterilisable part of the item to be sterilised within a container, the sterilisable part projecting from the container, and to form a seal around the item. The container and seal then serve to protect the non-sterilisable part from the sterilisation agent.

It is an object of the invention to provide a seal arrangement suitable for use in this application.

According to the invention there is provided a seal arrangement comprising a seal component engageable within an opening formed in a housing, the seal component defining an opening through which, in use, an item to be sealed to the housing extends, and a clamp device operable to apply a clamping load to the seal member, wherein the seal member is of split form, a seal keystone member being located in the split of the seal member.

It has been found that the provision of the keystone member enhances the integrity of the seal which can be formed.

Preferably, the clamp device applies a component of the clamping load in a direction which urges the keystone member into the split.

The invention will further be described, by way of example, with reference to the accompanying drawings, in which:
Figure 1 is a diagrammatic view of part of a sterilisation apparatus incorporating a seal arrangement in accordance with one embodiment of the invention;
Figure 2 is a diagrammatic view illustrating the seal arrangement; and
Figure 3 is a diagrammatic view illustrating the seal component thereof.

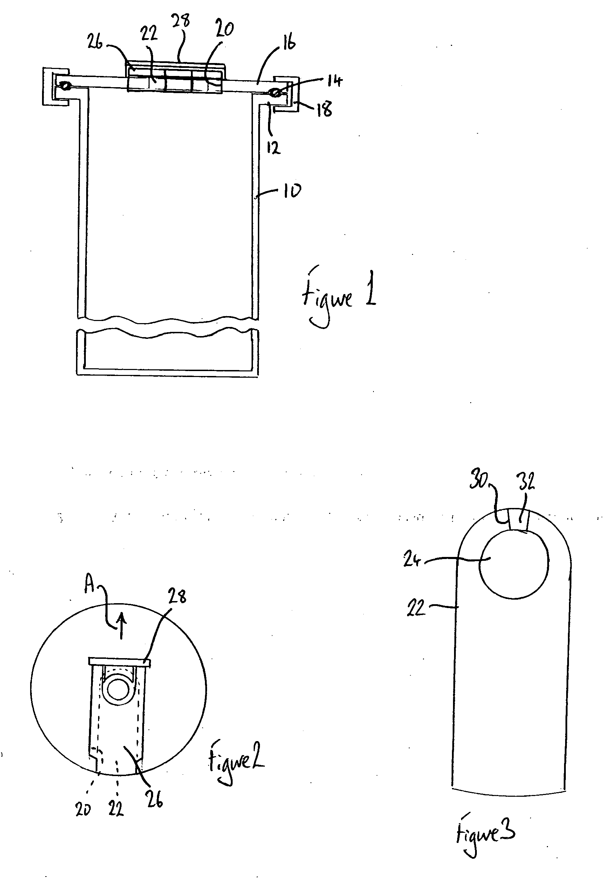

Figure 1 illustrates a sterilisation apparatus incorporating a seal arrangement in accordance with an embodiment of the invention. The sterilisation apparatus comprises a container 10, for example in the form of a cylinder, adapted to receive a non-sterilisable part of an item to be sterilised to protect if from a sterilising agent. The container 10 terminates, at its upper end, with an outwardly extending flange 12 formed with a recess serving to locate a seal ring 14. A plate 16, also formed with a recess arranged to accommodate the seal ring 14 is provided to close the container 10. A clamp device 18 clamps the plate 16 to the flange 12, compressing the seal ring 14 so as to form a substantially fluid-tight seal between the plate 16 and the flange 12.

The plate 16 is formed with a slot 20 extending from the periphery thereof towards the centre thereof. The slot 20 accommodates a seal component 22 of a resilient material. Although not illustrated, the slot 20 and seal component 22 are conveniently shaped to define inter-engaging tongue and groove like formations to locate the seal component 22 in the vertical direction in the orientation illustrated in Figure 1, thereby restricting vertical movement of the seal component 22. The seal component 22 is formed with an opening 24 through which, in use, an item to be sealed to the plate 16 and container 10 extends.

A clamp member 26 engages the upper surface of the seal component 22. The clamp member 26 is of forked form so as to extend around the opening 24, and a retainer 28 is provided on the plate 16 to receive the free ends of the clamp member 26. In use, an end of the clamp member 26 is engaged by the clamp device 18 and serves, in conjunction with the retainer 28, to apply a clamping load to the clamp member 26. The clamp member 26 and seal component 22 are of similar width, at the periphery of the plate 16, and are shaped, adjacent the periphery of the plate 16, such that the clamping load applied to the clamp member 26 results in the application of a clamping load to the seal member 22 acting in the direction of arrow A in Figure 2. This cooperation is achieved by virtue of the seal component 22 being tapered adjacent its free edge, and in the end part of the clamp member 26 being correspondingly angled.

As shown in Figure 3, the seal member 22 is shaped to define an opening 24. The seal member 22 is split along a split line 30, and a keystone seal member 32 of generally keystone or trapezoidal shape is located in the split 30. In the absence of the keystone member 32, the opening 24 would be of generally circular cross-sectional shape. The introduction of the keystone member 32 into the split 30 forces apart the edges of the split 30, therefore deforming the shape of the opening 24, and pre-stressing the seal material. Upon the application of the clamping load to the seal member 22 by the operation of the clamp member 26 and clamp device 18, the seal member 22 is compressed in the direction A. The shape of the keystone member 32 ensures that the keystone member 32 is retained within the split 30, when the compressive force is applied. It has been found that the provision of the keystone member enhances the integrity of the seal which can be formed, in use.

In use, the seal component 22 is arranged such that the article to be sterilised extends through the opening 24. Once so positioned, the seal member 22 is inserted into the slot 20 formed in the plate 16 and the plate 16 is positioned upon the container 10 with the seal ring 14 trapped therebetween. The clamp member 26 is positioned on top of the plate 16 and the clamp member 18 used to clamp the plate 16 and clamp member 26 in position. It will be appreciated that the application of the clamping load not only seals the plate 16 to the container 10, but also results in the application of a clamping load to the seal member 22 acting in direction A, compressing the seal member 22 and forcing the seal member 22 into sealing engagement both with the plate 16 and also around the item to be sealed.

Once sealed in this manner, air may be introduced into the container 10 and the container and article to be sterilised placed in a container containing a sterilising agent so as to sterilise the part of the item protruding from the container 10. After sterilisation, the clamp device 18 can be released to allow the plate 16 to be removed and allow the removal of the seal component 22 from the plate 16 thereby releasing the item from the plate 16. The air is conveniently introduced into the container through a valve (not shown) and, in the event that the seal leaks, air bubbles will form providing a visible indication that the seal is leaking. The operator can then remove and reseal the container 10.

It will be appreciated that a wide range of modifications and alterations may be made to the arrangement described hereinbefore without departing from the scope of the invention. For example, the seal component 22 may be adhered or otherwise secured to the underside of the clamp member 26. Further, if desired, guide rails or the like may be formed on the plate 16 to assist in the correct location of the clamp member 26 in position. The shapes of the various components may also be modified.

A range of other modifications and alterations may be made without departing from the scope of the invention. Further, the seal arrangement may be used in applications other than as described hereinbefore.

## Claims

1. A seal arrangement comprising a seal component engageable within an opening formed in a housing, the seal component defining an opening through which, in use, an item to be sealed to the housing extends, and a clamp device operable to apply a clamping load to the seal member, wherein the seal member is of split form, a seal keystone member being located in the split of the seal member.

2. A seal arrangement according to Claim 1, wherein the clamp device applies a component of the clamping load in a direction which urges the keystone member into the split.

3. A seal arrangement according to Claim 1 or Claim 2, wherein the housing forms part of a container provided with a gas valve to permit pressurisation of the container.

4. A seal arrangement substantially as hereinbefore described with reference to the accompanying drawings.

5. A sterilisation apparatus incorporating a seal arrangement as claimed in any of Claims 1 to 4.
